# EUROPEAN PATENT APPLICATION

(11) **EP 2 022 777 A2**
(43) Date of publication of application: **11.02.2009**
(21) Application number: 08010445.8
(22) Date of filing: 09.06.2008
(51) Int. Cl.: C07C 211/29, C07C 209/70

(54) **A process for the preparation of cinacalcet**

(30) Priority: 22.06.2007 IT MI20071261
(71) Applicant: Dipharma Francis S.r.l., 20021 Baranzate (MI) (IT)
(72) Inventor: Allegrini, Pietro, 20097 San Donato Milanese (MI) (IT); Attolino, Emanuele, 74019 Palagiano (TA) (IT)
(74) Representative: Minoja, Fabrizio

(57) **Abstract**

The invention provides a novel process for the preparation of a compound of formula **(I)** 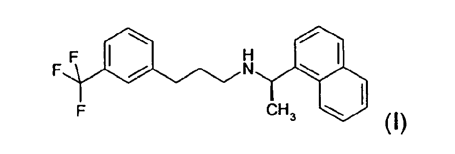 comprising the reduction of a compound of formula (II) or a salt thereof, in the presence of a catalyst, 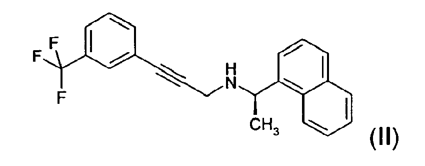 and novel intermediates useful for its synthesis.

## Description

### FIELD OF THE INVENTION

The present invention relates to a novel process for the preparation of (*R*)-(1-naphthalen-1-yl-ethyl)-[3-(3-trifluoromethyl-phenyl)-propyl]-amine, the salts thereof and novel intermediates useful for its synthesis.

### TECHNOLOGICAL BACKGROUND

(*R*)-(1-Naphthalen-1-yl-ethyl)-[3-(3-trifluoromethyl-phenyl)-propyl]-amine, i.e. cinacalcet, having formula (*I*) is a compound known for its anti-hyperparathyroid action, marketed as the hydrochloride.

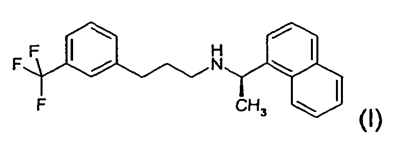

US 6,211,244 discloses its synthesis by condensation of 1-acetyl naphthalene and 3-[3-(trifluoromethyl)phenyl]propylamine in the presence of titanium isopropoxide and subsequent reduction of the resulting imine with sodium cyanoborohydride. The resulting racemic cinacalcet is then resolved by separation of the two optical antipodes with a chiral chromatographic column. "Drugs of the future", (2002), 27(9), page 831, reports a similar preparation of cinacalcet, starting from (R)-1-(1-naphthyl)ethylamine and 3-[3-(trifluoromethyl)phenyl]propionaldehyde, again by formation of the imine and reduction with sodium cyanoborohydride. US 6,211,244 also discloses another approach which involves the reduction of 3-(trifluoromethyl)-cinnamonitrile with diisobutylaluminium hydride (DIBAL-H) to yield the intermediate aluminium-imine, which affords cinacalcet by treatment with (R)-1-(1-naphthyl)ethylamine and sodium cyanoborohydride.

As it can be noted all of these processes either make use of toxic reagents (sodium cyanoborohydride) or starting materials difficult to prepare, or involve the resolution of a racemate with consequent remarkable increases in costs.

WO 2006/125026 suggests the synthesis of cinacalcet starting from 3-[3-(trifluoromethyl)phenyl]propanol, transformation of the hydroxyl function to a good leaving group to obtain an alkylating derivative and subsequent reaction of the latter with (R)-1-(1-naphthyl)ethylamine. In this case also, the preparation of the starting compound (3-[3-(trifluoromethyl)phenyl]propanol) requires at least 2 synthetic steps. Moreover, the process requires the use of a large excess of the alkylating agent originating from (3-[3-(trifluoromethyl)-phenyl]propanol) to obtain cinacalcet free from (R)-1-(1-naphthyl)ethylamine, which remarkably affects costs and production times.

There is therefore the need for an alternative synthesis, which can be easily applied to the preparation of cinacalcet or a salt thereof, and provides a high purity product while using comparatively inexpensive starting materials.

### DETAILED DISCLOSURE OF THE INVENTION

Object of the invention is a process for the preparation of a compound of formula (I), or a salt thereof, 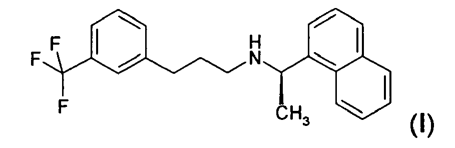 comprising the reduction of a compound of formula (II), or a salt thereof, in the presence of a catalyst, 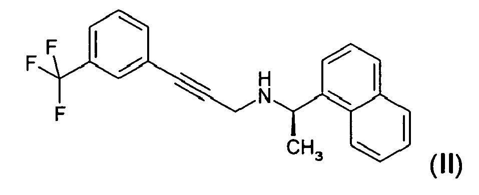 and, if desired, the conversion of a compound of formula (I) to a salt thereof, or vice versa.

A salt of a compound of formula (I) or (II) is for example an addition salt with a pharmaceutically acceptable organic or inorganic acid, preferably with hydrochloric acid.

The reduction reaction of a compound of formula (II), or a salt thereof, can be carried out for example by catalytic hydrogenation in the presence of a homogeneous or heterogeneous metal catalyst, for example based on Pd, Pt, Ni, Rh or Ru, preferably based on Pd. When the metal catalyst is heterogeneous, this is preferably deposited on an inert carrier, e.g. charcoal, barium hydroxide, alumina, calcium carbonate; preferably charcoal.

The concentration of the metal on carrier can range from about 1 to about 30%, preferably from about 5 to about 10%.

The hydrogen pressure used can range from about 1 atm to about 10 atm, the reaction is preferably carried out at atmospheric pressure.

The molar amount of the catalyst used to the compound of formula (II), or a salt thereof, approximately ranges from 0.1 to 10%, preferably from about 0.5 to about 5%.

The reaction can be carried out in the presence of an organic solvent, selected from e.g. a dipolar aprotic solvent, typically dimethylformamide, dimethylacetamide, acetonitrile, dimethylsulfoxide; an ether, e.g. tetrahydrofuran or dioxane or methyl-tert.butyl ether; a chlorinated solvent, e.g. dichloromethane; an apolar solvent, typically toluene or hexane; an alcohol, e.g. a C₁-C₆ alkanol, preferably a C₁-C₄ alkanol, in particular methanol, ethanol, isopropanol or butanol; an ester, e.g. ethyl acetate, isopropyl acetate, butyl acetate; a ketone, e.g. acetone, methyl-ethyl keto, methyl isobutyl keto; a carboxylic acid, e.g. acetic acid or propionic acid; or mixtures of two or more of said solvents, preferably 2 or 3. Alternatively, the reaction can be carried out in water or a mineral acid solution, for example hydrochloric acid or sulfuric acid, or mixtures thereof with one, two or three of the organic solvents mentioned above. The reaction can preferably be carried out in a C₁-C₆ alkanol or mixtures of more C₁-C₆ alkanols, preferably as exemplified above, or mixtures thereof with water, or an acetonitrile/water mixture; more preferably in isopropanol.

The reduction of a compound of formula (II), or a salt thereof, can also be carried out by hydrogen transfer reaction, using a homogeneous or heterogeneous metal catalyst, for example as defined above and in the same molar amount, and a hydrogen donor. The latter can be selected from the group comprising cyclohexene, cyclohexadiene, methylcyclohexene, limonene, dipentene, mentene, hydrazine, phosphinic acid or derivatives thereof, e.g. sodium hypophosphite, indoline, ascorbic acid, formic acid or sodium or ammonium salts thereof, and secondary C₁-C₆ alkanol, e.g. isopropanol; preferably cyclohexene or ammonium formate.

The molar ratio of the hydrogen donor to the compound of formula (II), or a salt thereof, can approximately range from 1.5 to 50, preferably from about 1.5 to about 10.

The reaction can be carried out in the presence of an organic solvent, selected from e.g. one of the solvents cited above or mixtures thereof with other solvents or with water, as mentioned above.

A compound of formula (I) can be converted to a salt thereof, or vice versa, according to known methods.

Preferably, the above reduction reactions are carried out using a salt of a compound of formula (II), more preferably the hydrochloride, to obtain directly the corresponding salt of a compound of formula (I), in particular the hydrochloride salt.

The resulting hydrochloride of the compound of formula (I) has purity higher than 99.5%, typically equal to or higher than 99.9%.

More particularly, the process of the invention includes a final step comprising the evaporation of the reaction solvent and the subsequent crystallization from a suitable solvent, for example from isopropanol, ethyl acetate or an acetonitrile/water mixture, thereby obtaining the hydrochloride salt of the compound of formula (I), having an XRPD substantially as reported in Figure 1 of WO 06/127833, corresponding to the Form I as therein defined, and crystal size characterized by a D₅₀ value approximately comprised between 25 and 250 µm.

In particular, the hydrochloride salt of compound of formula (I), having the same physical characteristics as reported above, can be obtained by carrying out the reduction reaction of the hydrochloride of compound of formula (II) in C1-C4 alkanol, e.g methanol or isopropanol, or an acetonitrile/water mixture, and carrying out the subsequent crystallization of the resulting product from a solvent, which may be the same reaction solvent or a different C1-C4 alkanol, optionally after concentration. Preferably the crystallization is carried out from isopropanol.

If desired, the D₅₀ value of the compound of formula (I), or of a salt thereof, can be reduced by micronisation or fine grinding, according to known methods.

A compound of formula (II), and the salts thereof, are novel and are an object of the present invention.

A compound of formula (II), or a salt thereof, can be prepared by a process comprising the reaction between a compound of formula (III) 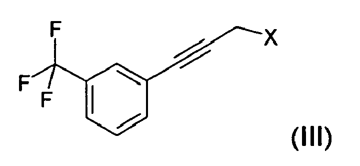 wherein X is a leaving group, and (R)-1-(1-naphthyl)ethylamine 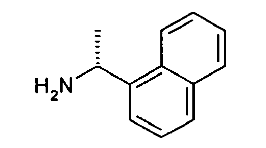 and, if desired, the conversion of a compound of formula (II) to a salt thereof.

A leaving group X is for example selected from a halogen atom, in particular chlorine, bromine or iodine; or an OSO₂R group, wherein R is for example an optionally substituted C₁-C₄ alkyl, phenyl or benzyl group, wherein the phenyl ring is in its turn optionally substituted; and N-imidazole. Preferably the leaving group is methyl, ethyl, trifluoromethyl, nonafluorobutyl, p-tolyl, p-bromobenzyl, p-nitrobenzyl; more preferably methyl.

The reaction between a compound of formula (III) and (R)-1-(1-naphthyl)ethylamine can be carried out according to known methods, in particular by treatment of (III) with an approximately equimolar amount of (R)-1-(1-naphthyl)ethylamine, in the presence of an organic or inorganic base, in an organic solvent or mixtures thereof.

An organic base is for example a tertiary amine, in particular triethylamine, diisopropylethylamine, diazabicycloundecene or diazabicyclooctane. An inorganic base is, for example, potassium carbonate.

An organic solvent can be for example a dipolar aprotic solvent, typically dimethylformamide, dimethylacetamide, acetonitrile, dimethylsulfoxide; an ether, typically tetrahydrofuran or dioxane or methyl-tert.butyl ether; a chlorinated solvent, typically dichloromethane; an apolar solvent, typically toluene or hexane; an ester, typically ethyl acetate, isopropyl acetate, butyl acetate; a ketone, typically acetone, methyl-ethyl ketone, methyl isobutyl ketone; or mixtures of two or more of said solvents, preferably 2 or 3.

A compound of formula (I) or (II) can be converted to a salt thereof by reaction with an organic or inorganic acid, preferably hydrochloric acid, in water or an organic solvent as herein defined, or mixtures thereof. The acid can be used neat or in aqueous solution. Salification is preferably carried out with aqueous hydrochloric acid in solution of concentration approximately ranging from 10 to 37%.

A compound of formula (III) can be obtained according to known methods, for example by reaction of a compound of formula (IV) 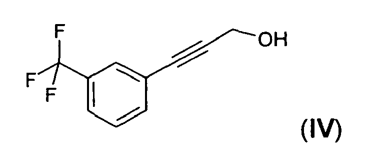 with methanesulfonyl chloride in the presence of an organic base and of a solvent, as indicated above with reference to the preparation of a compound of formula (II).

The compound of formula (IV) is known and can be prepared by known methods, for example by reaction of a compound of formula (V) 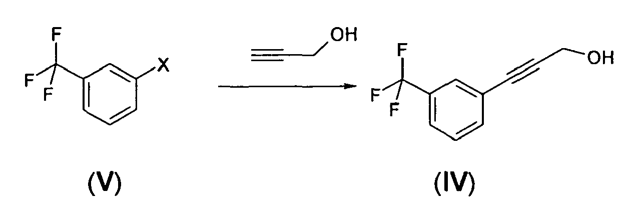 wherein X is as defined above, with propargyl alcohol, in the presence of a catalyst, e.g. a Pd(II) salt, in particular PdCl₂ or Pd(OAc)₂ and a base, typically an inorganic or organic base, in particular a secondary or tertiary amine; optionally in the presence of Cul, a ligand, e.g. triphenylphosphine, and a solvent, e.g. an organic solvent as defined above.

The compounds of formula (III) are novel and are a further object of the present invention.

The compound of formula (V) are known and commercially available.

The following examples illustrate the invention.

### Example 1.

### Synthesis of compound (IV): 1-(3-Hydroxy-prop-1-inyl)-3-trifluoromethyl-benzene

50 g of 3-bromo benzotrifluoride (0.22 mole, 31 ml) are dissolved in 75 ml of triethylamine and 25 ml of dimethylacetamide under nitrogen atmosphere. The mixture is heated to 50°C, then 340 mg (1.76 mmoles) of copper (I) iodide, 155 mg (0.88 mmoles) of palladium (II) chloride and 930 mg (3.55 mmoles) of triphenylphosphine are added. The mixture is adjusted to 70°C and 16 ml (16 g, 0.29 moles) of propargyl alcohol are slowly dropped therein. After 17 hours, the reaction mixture is diluted with toluene and filtered. The filtrate is washed in succession with a 1 N HCl aqueous solution, saturated NaHCO₃ and water. The organic phase is then dried over dry Na₂SO₄, and filtered. The solvent is evaporated off under reduced pressure to yield compound (IV).

¹H NMR (300 MHz, DMSO-d6), ppm: 7,72-7,69 (m, 3H), 7,60 (t, 1H, *J* 7,5 Hz), 5,38 (t, 1 H, *J* 6,0 Hz), 4.31 (d, 2H, *J* 6,0 Hz).

### Example 2.

### Synthesis of a compound (III): 1-(3-Methanesulfonyloxy-prop-1-inyl)-3-trifluoromethyl-benzene

44 g of compound of formula (IV) (0.22 moles) and 36.8 ml of triethylamine are dissolved in 195 ml of toluene. The mixture is cooled in an ice bath and a solution of methanesulfonyl chloride (18.7 ml, 0.24 moles) in toluene (30 ml) is slowly dropped therein. After completion of the addition, the mixture is brought again at room temperature and filtered. The solution is washed with a NaHCO₃ saturated solution, dried over dry Na₂SO₄ and filtered. The solvent is evaporated under reduced pressure to yield the compound III.

¹H NMR (300 MHz, DMSO-d6), ppm: 7.84-7.74 (m, 3H), 7.64 (t, 1 H, J 7.8 Hz), 5.21 (s, 2H), 3.28 (s, 3H).

### Example 3.

### Synthesis of compound of formula (II) hydrochloride: (1-Naphthalen-1-yl-ethyl)-[3-(3-trifluoromethyl-phenyl)-prop-2-inyl]-amine

5.9 ml (6.2 g, 0.036 mmoles) of (R)-1-(1-naphthyl)ethylamine are dissolved in acetonitrile (30 ml). Then 4.97 g (0.036 mmoles) of K₂CO₃ and a solution of compound of formula (III) (10.1 g, 0.036 mmoles) in acetonitrile (15 ml) are added. The reaction mixture is heated to 50°C and kept under stirring for 17 hours, then concentrated under reduced pressure. The residue is diluted in toluene and filtered. The solution is heated to 50°C and treated with 1M HCl. The suspension is filtered and the resulting precipitate is crystallized from a toluene/methanol solution. The hydrochloride of compound of formula (II) is then dried, and it has purity higher than 99.5%.
¹H NMR (300 MHz, DMSO-d6), ppm: 8.32 (d, 1 H, J 9.0 Hz), 8.05-7.96
(m, 3H), 7.80-7.50 (m, 7H), 5.53 (q, 1 H, J 6.6 Hz), 4.15, 4.00 (system AB, 2H, J 17.1 Hz), 1.73 (d, 2H, J 6.6 Hz).

### Example 4.

### Synthesis of compound of formula (I) hydrochloride: cinacalcet

34.2 g of compound of formula (II) hydrochloride (87.7 mmoles) are dissolved in 730 ml of isopropanol containing 7.40 g of 5% Pd/C (containing 49.4% water). The mixture is treated with hydrogen under atmospheric pressure at room temperature for 3 hours and subsequently filtered through Celite®. The clear solution is concentrated at 70°C under reduced pressure until slight turbidity, then left to slowly cool to 10°C. Cinacalcet hydrochloride crystals are washed with water and dried, to afford 30.7 g of product in 90% yield and with purity higher than 99.5%. The resulting product has an XRPD substantially as reported in Figure 1 of WO 06/127833, corresponding to the Form I as therein defined, and crystal size characterized by a D₅₀ value approximately between 25 and 250 µm.

¹H NMR (300 MHz, DMSO-d6), ppm: 9.80 (bs, 1 H), 9.30 (bs, 1 H), 8.22 (d, 1 H, J 7.8 Hz), 8.02-7.94 (m, 3H), 7.62-7.44 (m, 7H), 5.28 (q, 1 H, J 6.6 Hz), 2.96-2.92 (m, 1 H), 2.78-2.66 (m, 3H), 2.04-1.95 (m, 2H), 1.67 (d, 2H, J 6.6 Hz).

### Example 5.

### Synthesis of compound of formula (III): 1-(3-Methanesulfonyloxy-prop-1-inyl)-3-trifluoromethyl-benzene

3-Bromo benzotrifluoride (50 g, 0.22 mol) is dissolved in a mixture of triethylamine (75 ml) and dimethylacetamide (25 ml) under nitrogen, then cuprous iodide (340 mg, 1.76 mmol), palladium chloride (155 mg, 0.88 mmol) and triphenylphosphine (930 mg, 3.55 mmol) are added. The mixture is heated to 70-75°C and propargyl alcohol (16 g, 0.29 moli) is slowly added. After stirring at 70-75°C for 8 h, the reaction mixture is cooled to room temperature and diluted with toluene (125 ml) and water (75 ml). The biphasic system is neutralized by treating with HCl 37%. After separation the organic phase is washed first with a diluted solution of ammonia and then with water. After filtration and separation, the organic phase is concentrated at reduced pressure. The residue is diluted in toluene (300 ml) and diisopropylethylamine (29.8 g, 0.23 mol) is added. The solution is cooled to -5-10°C temperature and methanesulfonyl chloride (25.2 g ml, 0.22 moles) is slowly dropped therein. After completion of the addition (3h), the mixture is neutralized by slow addition of a diluted solution of sulfuric acid. The organic phase is separated, washed with water, filtered and concentrated under reduced pressure to yield 55.1 g of compound (III) in a 90% yield.

### Example 6.

### Synthesis of compound of formula (II) hydrochloride: (1-Naphthalen-1-yl-ethyl)-[3-(3-trifluoromethyl-phenyl)-prop-2-inyl]-amine

Compound (III), obtained in Example 5, (50.1 g, 0.18 moles) is dissolved in acetonitrile (500 ml) and the resulting solution is treated with (R)-1-(1-naphthyl)ethylamine (92.5 g, 0.54 moles). The solution is stirred at 25°C for 16-20 h and then concentrated at reduced pressure. The residue is diluted with toluene and water and acidified to pH 5 with diluted HCl at 40-45°C. The organic phase is separated and concentrated at reduced pressure and the residue is diluted in isopropanol and acidified to pH 1 by adding concentrated HCl. The mixture is heated to 70°C and then slowly cooled. The crystalline solid is filtered off, washed with isopropanol and dried yielding 53 g of compound (II) hydrochloride with a purity higher than 99%.

## Claims

1. A process for the preparation of a compound of formula (I), or a salt thereof, 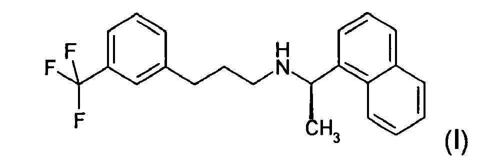 comprising the reduction of a compound of formula (II), or a salt thereof, in the presence of a catalyst, 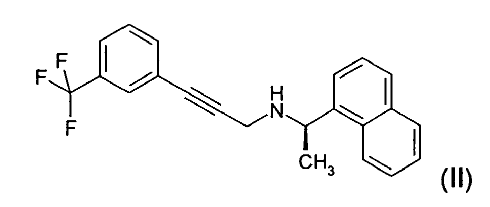 and, if desired, the conversion of a compound of formula (I) to a salt thereof, or vice versa.

2. The process according to claim 1, wherein the reduction is carried out by catalytic hydrogenation in the presence of a homogeneous or heterogeneous metal catalyst.

3. The process according to claim 1, wherein the reduction is carried out by hydrogen transfer reaction, using a homogeneous or heterogeneous metal catalyst and a hydrogen donor.

4. The process according to claims 2 and 3, wherein the metal catalyst is based on Pd, Pt, Ni, Rh or Ru and is deposited on an inert carrier.

5. The process according to claim 4, wherein the concentration of the metal catalyst on the inert carrier approximately ranges from 1 to 30%.

6. The process according to claim 2 or 4, wherein the reduction is carried out under a hydrogen pressure approximately ranging from 1 atm and 10 atm.

7. The process according to claim 2 or 3, wherein the molar ratio of catalyst to compound of formula (II), or a salt thereof, approximately ranges from 0.1 to 10%.

8. The process according to claim 3, wherein the hydrogen donor is selected from cyclohexene, cyclohexadiene, methylcyclohexene, limonene, dipentene, mentene, hydrazine, phosphinic acid or a derivative thereof, indoline, ascorbic acid, formic acid or a sodium or ammonium salt thereof, and a secondary C₁-C₆ alkanol.

9. The process according to claim 7 or 8, wherein the molar ratio of hydrogen donor to compound of formula (II), or a salt thereof, approximately ranges from 1.5 to 50.

10. The process according to claim 1, wherein the reaction is carried out in an alkanol, a mixture of more alkanols, a mixture thereof with water, or an acetonitrile/water mixture.

11. The process according to any one of the preceding claims, wherein the compound of formula (II) is in the salified form.

12. The process according to claim 1, wherein the hydrochloride salt of the compound of formula (I), is obtained by carrying out the reduction reaction of the hydrochloride of compound of formula (II) in C1-C4 alkanol or in acetonitrile/water mixture, and the subsequent crystallization of the resulting product is from carried out from a solvent, which may be the same reaction solvent or a different C1-C4 alkanol, optionally after concentration.

13. The process according to claim 12, wherein the crystallization is carried out from isopropanol.

14. A compound of formula (II), or a salt thereof, or of formula (III) 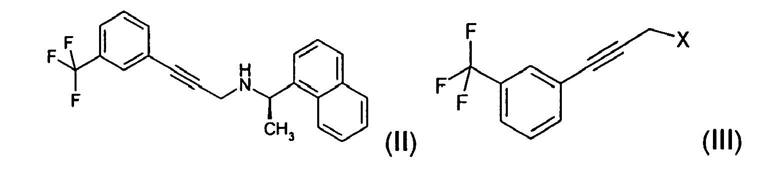 wherein X is a leaving group.
